(19) Europäisches Patentamt
European Patent Office
Office européen des brevets

(11) Numéro de publication : **0 163 560 B1**

(12) **FASCICULE DE BREVET EUROPÉEN**

(45) Date de publication du fascicule du brevet : **09.08.89**

(51) Int. Cl.$^4$ : **C 01 B 3/26**, B 01 J 29/02, C 10 G 11/05

(21) Numéro de dépôt : **85400781.2**

(22) Date de dépôt : **19.04.85**

(54) **Nouvelle zéolite à structure bidimensionnelle et application de ladite zéolite.**

(30) Priorité : **25.04.84 FR 8406482**

(43) Date de publication de la demande : **04.12.85 Bulletin 85/49**

(45) Mention de la délivrance du brevet : **09.08.89 Bulletin 89/32**

(84) Etats contractants désignés : **BE DE FR GB IT NL**

(56) Documents cités :
**EP-A- 0 073 718**
**EP-A- 0 110 628**
**US-A- 4 060 480**
**US-A- 4 176 090**
**US-A- 4 216 188**
**Scheffer/Schachtschabel, "Lehrbuch der Bodenkunde",10 Auflage 1979, Ferdinand Euke Verlag Stuttgart page 41**
**Le dossier contient des informations techniques présentées postérieurement au dépôt de la demande et ne figurant pas dans le présent fascicule.**

(73) Titulaire : **COMPAGNIE DE RAFFINAGE ET DE DISTRIBUTION TOTAL FRANCE**
**84, rue de Villiers**
**F-92300 Levallois Perret (FR)**

(72) Inventeur : **Plee, Dominique**
**36, Avenue Paul-Vaillant Couturier**
**F-78190 Trappes (FR)**
Inventeur : **Schutz, Alain**
**Rampe du Couvent No 9 Boîte 6**
**B-1348 Louvain La Neuve (BE)**
Inventeur : **Borg, Françoise**
**Rue de l'Abbaye**
**F-27170 Beaumontel (FR)**
Inventeur : **Poncelet, Georges**
**15, rue de Wavre**
**B-5998 Beauvechain (BE)**
Inventeur : **Jacobs, Pierre**
**Streland Straat 104**
**B-1686 Gooik (BE)**
Inventeur : **Gatineau, Lucien**
**1, Place du Val**
**F-45100 Orléans-La-Source (FR)**
Inventeur : **Fripiat, José**
**31 La Tuillerie Marcilly en Vilette**
**F-45240 La Ferté-Saint-Aubin (FR)**

(74) Mandataire : **Jolly, Jean-Pierre et al**
**Cabinet BROT et JOLLY 83, rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne un nouveau produit, appelé zéolite à structure bidimensionnelle, obtenu à partir d'une argile et son application à la conversion de charges d'hydrocarbures.

Certaines argiles ont une structure gonflante. Elles ont la propriété de pouvoir adsorber, de l'eau notamment, entre les différents feuillets qui la composent. C'est le cas en particulier des argiles du groupe des smectites. Ces argiles ont une structure que l'on peut représenter, d'une manière simplifiée, comme composée de feuillets à couches triples comportant deux couches de tétraèdres $SiO_4$ et une couche intermédiaire dioctaédrique ou trioctaédrique, ces octaèdres ayant pour formule $MX_6$; M pouvant être un ion trivalent (argile dioctaèdrique) ou divalent (argile trioctaèdrique) avec X = O et OH.

La cohésion entre les couches est assurée par la mise en commun d'atomes d'oxygène apicaux. Les atomes de silicium des tétraèdres peuvent être remplacés en partie par des atomes d'aluminium. C'est le cas notamment dans la beidellite.

De la même façon, dans les octaèdres de la couche intermédiaire, les atomes d'aluminium peuvent être remplacés en partie par des atomes de magnésium ou de fer dans le cas des smectites dioctaèdriques ; dans les smectites trioctaèdriques, les atomes de magnésium de la couche intermédiaire peuvent être remplacés en partie par des atomes de fer et de lithium.

Il est bien connu que la surface spécifique d'un support a une très grande importance en catalyse et ces argiles, du fait de leur propriété d'être expansibles, pourraient être utilisées comme catalyseurs ou supports de catalyseurs, notamment pour la conversion d'hydrocarbures. Mais, une fois expansées, c'est-à-dire après adsorption de l'eau entre leurs différents feuillets, ces argiles ont l'inconvénient de perdre leur caractère expansé par chauffage à 100 °C, et, de ce fait, de ne pas conserver l'augmentation de surface spécifique pouvant résulter de leur expansion.

On peut rappeler que l'état d'expansion d'une argile est défini par l'espacement interfoliaire et l'espacement basal, qui sont mesurés par diffraction de rayons X.

L'espacement interfoliaire est, comme son nom l'indique, l'espacement entre deux feuillets. A l'état non expansé, après séchage de l'argile, il est donc nul.

L'espacement basal, représenté par le sigle $d_{001}$, est défini comme la somme de l'épaisseur d'un feuillet et de l'espacement interfoliaire.

Dans le cadre d'une smectite, l'épaisseur d'un feuillet est de 9,6 Angströms. A l'état expansé, l'espacement interfoliaire peut dépasser environ 10 Angströms et l'espacement basal peut donc dépasser environ 20 Angströms.

On a cherché, dans le but notamment d'utiliser les argiles expansibles comme supports de catalyseurs ou comme catalyseurs, à obtenir des argiles ayant un espacement basal aussi élevé que possible, cet espacement basal pouvant être maintenu quand l'argile est soumise à un traitement thermique.

On a ainsi réussi à introduire entre les feuillets d'argile des piliers ou ponts pour obtenir des argiles qui seront désignées, dans la suite de la présente description, par le terme « argiles pontées ».

Une méthode bien connue consiste à introduire, entre les feuillets d'argile, des ponts constitués par des oligomères d'un hydroxyde d'un métal et, notamment, d'un hydroxyde d'aluminium.

Cette méthode, notamment décrite dans l'Article de MM. Lahav, U. Shani et J. Shabtai, paru dans Clays and Clays Mineral, Volume 26, numéro 2, pages 107 à 115 (1978), consiste à mettre en contact un oligomère d'hydroxyde d'aluminium avec une suspension aqueuse diluée de montmorillonite.

La montmorillonite séparée du mélange est ensuite lavée par de l'eau.

Une autre méthode, décrite dans le brevet français nº 2 512 043 consiste à préparer également un mélange d'une suspension aqueuse d'argile et d'un oligomère d'hydroxyde d'aluminium, notamment, et ensuite à dialyser ce mélange. Les argiles pontées ainsi obtenues sont utilisées comme catalyseurs ou supports de catalyseurs.

Une autre méthode, décrite dans le brevet français nº 2 394 324, consiste à mettre en contact l'argile avec une solution de chlorohydroxyde d'aluminium appelé « chlorhydrol ». L'argile peut-être notamment une beidellite. Les argiles pontées ainsi préparées peuvent servir de catalyseurs de conversion de charges d'hydrocarbures. Ce brevet ne donne cependant aucun exemple d'utilisation d'une beidellite pontée comme catalyseur.

L'article de G.W. Brindley et R.E. Sempels, paru dans Clays Mineral, Volume 2, pages 229 à 237 (1977), décrit également la préparation de beidellites pontées, à partir d'une beidellite de Taiwan, par une méthode semblable à celle décrite dans l'article de Lahav, U. Shani et J. Shabtai, cité précédemment.

Enfin, US-A-4 176 090 décrit un procédé de préparation d'une argile pontée, qui comprend un traitement d'une smectite avec un complexe d'aluminium ou de zirconium à une température comprise entre 5 et 200 °C, pendant 0,1 à 4 heures, et éventuellement un traitement thermique du produit obtenu, variant, suivant la smectite, entre 200 et 700 °C. Dans un exemple concernant une beidellite, aucun traitement thermique n'est mentionné.

La Demanderesse a maintenant découvert que l'on pouvait préparer des produits d'une structure particulière à partir d'argiles comportant des substitutions en couche tétraédrique. Par « substitutions en couche tétraédrique » on entend que des atomes de silicium sont remplacés par des atomes d'aluminium. Cette préparation a été rendue possible, comme il sera décrit plus loin, en faisant suivre la préparation

d'une beidellite pontée, dans des conditions connues en soi, par un traitement thermique approprié. La structure particulière de ce produit est déterminée grâce à l'analyse par résonance magnétique nucléaire à haute résolution. Pour le différencier des beidellites pontées précédemment décrites, la Demanderesse l'a appelé zéolite à structure bidimensionnelle. La nature différente de ce produit par rapport aux argiles à substitution en couche octaédrique pontées déjà connues est confirmée par des propriétés catalytiques meilleures.

Le but de la présente invention est donc de préparer, à partir d'une argile à substitution en couche tétraédrique, un produit ayant des propriétés catalytiques intéressantes.

A cet effet, l'invention a pour objet, à titre de produit nouveau, une zéolite à structure bidimensionnelle présentant un spectre distinctif de résonance magnétique nucléaire, obtenue par un procédé comportant les étapes suivantes :

a) le traitement d'une argile du type beidellite dans laquelle des atomes de silicium ont été remplacés par des atomes d'aluminium dans les couches tétraédriques, sous forme d'une suspension aqueuse, par une solution aqueuse contenant au moins un hydroxyde d'au moins un métal ;

b) l'élimination de l'excès de l'hydroxyde du métal n'ayant pas réagi sur l'argile, ladite élimination conduisant, après séchage de l'argile traitée, à l'obtention d'un précurseur de la zéolite ;

c) le traitement thermique du précurseur de la zéolite, ledit traitement conduisant à l'obtention de la zéolite à structure bidimensionnelle ;

caractérisée en ce que ledit traitement thermique du précurseur a été effectué à une température comprise entre 250 et 450 °C.

L'invention a également pour objet l'utilisation des zéolites à structure bidimensionnelle selon l'invention, comme catalyseurs ou comme supports de catalyseurs, notamment pour la conversion de charges d'hydrocarbures.

Les argiles comportant des substitutions d'atomes de silicium par des atomes d'aluminium en couche tétraédrique, utilisables pour la préparation des zéolites à structure bidimensionnelle, selon l'invention, peuvent être des argiles naturelles ou synthétiques. La méthode de préparation de beidellites synthétiques utilisée par la Demanderesse est inspirée des articles de V.A. Frank-Kamenetskiy, N.V. Kotov et A.N. paru dans Geokhimiya, numéro 8, pages 1153 à 1162 (1973) et de D. Eberl et J. Hower paru dans Clays and Clays mineral, Volume 25, pages 215 à 227 (1977).

Cette méthode consiste à préparer, à partir de sels d'aluminium et de silicium, un gel contenant de l'alumine et de la silice. Ce gel est ensuite traité dans des conditions hydrothermales en présence de soude pour obtenir la beidellite. Des exemples de préparation de beidellites seront donnés plus loin.

L'argile à substitutions tétraédriques peut également être obtenue par modification d'une argile telle que la montmorillonite, ne comportant pas de telles substitutions.

La présence de substitutions tétraédriques dans des argiles peut-être déterminée selon une méthode décrite dans l'article de MM. B. Chourabi et J. J. Fripiat, paru dans Clays and Clays mineral, Volume 29, numéro 4, pages 260 à 268 (1981), intitulé « Determination of tetrahedral heterogeneity from vibrational spectra of ammonium in smectites ». La substitution tétraédrique est caractérisée, à l'analyse infrarouge, par l'apparition d'une bande de vibration à 3030 $cm^{-1}$ de l'ion ammonium en position sur un site d'échange de l'argile.

On peut ensuite, en étudiant l'argile synthétisée par diffraction de rayons X, vérifier qu'il n'y a qu'une seule phase cristalline.

La solution aqueuse d'au moins un hydroxyde d'au moins un métal, utilisée pour le traitement de l'argile, est une solution contenant des ions $OH^-$ et des ions métalliques $M^{x+}$ dans laquelle le rapport $OH^-/M^{x+}$ est tel que le seuil de précipitation de l'hydroxyde n'est pas atteint. Il est important que la solution d'hydroxyde soit limpide avant son utilisation. La clarification de la solution d'hydroxyde peut-être obtenue par chauffage ou vieillissement.

Selon l'invention, l'hydroxyde peut-être choisi dans le groupe formé par les hydroxydes des métaux choisis dans le groupe formé par les hydroxydes de l'aluminium, du nickel, du cobalt, du vanadium, du molybdène, du rhénium, du fer, du cuivre, du ruthénium, du chrome, du lanthane, du cérium, du titane, du bore, du gallium et les hydroxydes mixtes de ces éléments.

La Demanderesse a utilisé avec succès des solutions d'hydroxyde d'aluminium. Par solution d'hydroxyde d'aluminium, on désigne une solution contenant des monomères ou des oligomères aluminiques.

Dans le procédé selon l'invention, l'hydroxyde peut-être obtenu par action d'une base sur une solution d'un sel d'un métal dans laquelle le métal se trouve sous forme de cation, ou d'un acide sur une solution d'un sel d'un métal dans laquelle le métal se trouve sous forme d'anion.

Le mélange de la solution d'au moins un hydroxyde d'un métal et de la suspension d'argile peut-être réalisé de différentes façons :

soit en préparant la solution d'hydroxyde, par exemple par action d'une base sur un sel, puis en ajoutant la solution obtenue à la suspension d'argile.

soit en ajoutant à la suspension d'argile une solution du sel du métal, puis en ajoutant une solution de base.

soit encore en ajoutant simulténément à la suspension d'argile une solution du sel du métal et une base.

Autrement dit, la solution d'hydroxyde peut-être préparée avant son mélange avec la suspension d'argile, ou en ajoutant à la suspension d'argile les précurseurs de la solution d'hydroxyde.

La solution d'hydroxyde peut-être également obtenue à partir de certains composés qui s'hydrolysent spontanément, sans que l'on ait besoin de rajouter une base, par exemple à partir des oxychlorures de zirconium, de vanadium, de molybdène, de rhénium, de titane et de leurs combinaisons.

Dans le procédé selon l'invention, un des paramètres importants est le rapport des ions $OH^-/M^x$ dans la solution d'hydroxyde où x définit le degré d'oxydation du métal.

Quand x = I, le rapport $OH^-/M^I$ peut-être compris entre 0,2 et 1, de préférence entre 0,2 et 0,8 et, mieux encore, entre 0,3 et 0,7.

Quand x = II, le rapport $OH^-/M^{II}$ peut-être compris entre 0,2 et 2, de préférence entre 0,4 et 1,8 et, mieux encore, entre 0,5 et 1,4.

Quand x = III, le rapport $OH^-/M^{III}$ peut-être compris entre 0,3 et 3, de préférence entre 0,6 et 2,4 et, mieux encore, entre 0,8 et 1,8.

Quand x = IV, le rapport $OH^-/M^{IV}$ peut-être compris entre 0,4 et 4, de préférence entre 0,8 et 3,6 et, mieux encore, entre 1 et 2,8.

Dans le procédé selon l'invention, la concentration, dans le mélange de la suspension d'argile et de la solution d'hydroxyde, de l'ion du métal utilisé pour former les piliers d'oxydes métalliques doit être telle qu'elle soit suffisamment importante pour qu'il y ait formation du précurseur de la zéolite et de ne pas être trop élevée pour qu'il n'y ait pas trop d'oxydes métalliques insérés entre les feuillets d'argile, de façon à ne pas nuire à la porosité de l'argile.

Elle peut-être comprise, par exemple, exprimée en milliéquivalents du métal considéré par gramme d'argile, entre 2 et 60 et, de préférence, entre 12 et 30.

La concentration en argile dans le mélange final, c'est-à-dire après mélange de la solution d'hydroxyde et de la suspension d'argile initiale, doit être telle qu'elle soit suffisamment importante pour ne pas avoir à manipuler de grands volumes de mélange, et ne pas être trop élevée, car, si le mélange est trop concentré en argile, il est difficile à manipuler.

La concentration en argile du mélange final peut être par exemple comprise entre 0,1 et 4 % en poids et, de préférence, comprise entre 0,5 et 3 % en poids.

Après le traitement de l'argile par l'hydroxyde métallique, l'excès d'hydroxyde n'ayant pas réagi sur l'argile est éliminé.

Cette élimination peut-être effectuée en séparant l'argile du mélange réactionnel et en la lavant avec de l'eau.

On peut également effectuer la dialyse du mélange de la solution d'au moins un hydroxyde d'un métal et de la suspension d'argile.

Cette dialyse peut-être effectuée en plaçant ledit mélange dans une enceinte constituée par une membrane semi-perméable choisie dans le groupe constitué par les membranes à base de cellulose régénérée ou toute autre membrane semi-perméable.

L'enceinte est ensuite plongée dans de l'eau, de préférence de l'eau déminéralisée, bien que toute eau (distillée, eau de ville) puisse convenir, du moment qu'elle a une force ionique inférieure à celle de l'eau contenue dans la membrane de dialyse.

Cette opération peut-être effectuée en discontinu ou en continu, c'est-à-dire, dans ce dernier cas, en plaçant l'enceinte dans un courant d'eau.

L'opération de dialyse peut-être effectuée à la température ambiante, mais peut-être activée en élevant la température de l'eau. La température limite est fixée par la température d'ébullition de l'eau à la pression à laquelle l'opération est effectuée.

L'argile traitée ne contenant plus d'excès d'hydroxyde métallique est ensuite séchée, par exemple par lyophilisation. On peut ainsi obtenir le précurseur de la zéolite.

Le précurseur de la zéolite est ensuite soumis à un traitement thermique à une température comprise entre 250 et 450 °C.

Après ce traitement, on obtient la zéolite bidimensionnelle selon l'invention.

Les zéolites à structure bidimensionnelle selon l'invention sont caractérisées par leur spectre de résonance magnétique nucléaire à haute résolution. L'étude a été effectuée par la Demanderesse sur un appareil d'une fréquence de 500 Mégahertz (résonance du proton).

On sait qu'un noyau atomique de spin I développe 2I + 1 niveaux d'énergie lorsqu'il est placé dans un champ magnétique. Selon les règles de sélection, si seule l'interaction Zeeman était impliquée, les niveaux seraient également espacés et une seule fréquence de résonance devrait être observée. Elle correspondrait à une transition m = + 1, par exemple + 1/2, — 1/2 ou 1/2, 3/2 etc...

Lorsque les noyaux ne sont pas infiniment séparés et que, de plus, le solide contient des noyaux de types différents, ce qui est le cas des zéolites à structure bidimensionnelle selon l'invention, les interactions magnétiques provoquent des modifications dans la position des niveaux d'énergie, ce qui amène l'apparition de raies nouvelles.

Pour le noyau d'un atome de silicium de masse atomique 29, de spin 1/2, l'effet principal est dû à un écrantage de champ magnétique principal à l'emplacement du noyau. Ceci produit une raie de résonnance large due à ce que l'on appelle l'anisotropie du déplacement chimique. En faisant tourner l'échantillon autour de l'angle magique, qui est celui qui annule la fonction ($3\cos^2 \theta$-1) où $\theta$ est l'angle entre

le moment magnétique et le champ magnétique directeur, on fait disparaître les causes de l'élargissement de la raie et on obtient une raie fine qui se trouve à une position qui sera fonction du champ magnétique local ressenti par le noyau.

La Demanderesse a utilisé cette technique de rotation autour de l'angle magique pour caractériser les zéolites à structure bidimensionnelle selon l'invention. Elle a également étudié, en comparaison, d'autres argiles pontées, pour montrer les différences qui existent entre ces produits et ceux de l'invention.

Ces études, qui seront décrites plus loin dans les exemples, permettent de détecter l'environnement des atomes de silicium et d'aluminium dans les argiles modifiées par un traitement par un hydroxyde métallique, et d'en tirer des informations essentielles sur les ordres à courte distance dans ces solides.

Les spectres de résonance des noyaux d'un atome de silicium de masse atomique 29, $^{29}Si$, et d'un atome d'aluminium de masse atomique 27, $^{27}A1$, d'un solide, constituent une véritable carte d'identité, au même titre qu'un diagramme de diffraction des rayons X qui établit l'ordre à longue distance. Cette dernière technique, pour les argiles pontées, ne fournit que l'espacement basal, qui, dans tous les cas, est de l'ordre de 18 Angströms. L'étude aux rayons X ne permet pas de différencier les argiles pontées et ne donne aucune indication sur l'organisation des liens Si-O-Al qui sont à l'origine des propriétés catalytiques de ce type de solide.

Les zéolites à structure bidimensionnelle selon l'invention ont une très bonne stabilité thermique et conservent leur espacement basal quand elles sont portées à haute température. Elles peuvent donc servir de supports de catalyseurs et de catalyseurs, notamment dans la conversion d'hydrocarbures, en présence, ou non, d'hydrogène.

Elles peuvent servir, en particulier, de supports de catalyseurs ou de catalyseurs pour l'isomérisation et/ou le craquage d'hydrocarbures, comme par exemple le craquage de charges lourdes d'hydrocarbures provenant de la distillation du pétrole brut ayant un point d'ébullition supérieur à 300 °C.

Pour la préparation de ces catalyseurs à partir de ces zéolites, on peut déposer sur celles-ci au moins un des éléments des groupes IA, IIA, IIIA, IVA, VA, VIA, VIIA, VIII, IB, IIB, IIIB, IVB, VB et VIIB de la classification périodique des éléments.

Les catalyseurs peuvent contenir, notamment, rapporté au poids total du catalyseur, de 0,02 à 3 % et de préférence de 0,10 à 1,5 % d'au moins un métal de la mine du platine.

Par métal de la mine du platine, on entend l'un des métaux suivants :

le ruthénium
le rhodium
le palladium
l'osmium
l'iridium
le platine.

L'invention est illustrée à titre non limitatif, par les cinq exemples qui suivent.

Les cinq Figures jointes à la présente description seront explicitées dans ces exemples.

## Exemple 1

Cet exemple concerne la préparation :

de sept argiles comportant des substitutions aluminium en couche tétraèdrique AST1, AST2, AST3, AST4, AST5, AST6 et AST7.

à partir de ces argiles, de douze zéolites à structure bidimensionnelle selon l'invention ZSB11, ZSB12, ZSB21, ZSB22, ZSB31, ZSB32, ZSB41, ZSB42, ZSB51, ZSB52, ZSB61 et ZSB71,

à partir d'une argile naturelle de Taïwan d'une néolite à structure bidimensionnelle selon l'invention ZSB81,

d'une montmorillonite pontée témoin MPT,
d'une hectorite pontée témoin HPT,
d'une laponite pontée témoin LPT.

### Préparation des argiles comportant des substitutions en couche tétraèdrique

On a préparé sept argiles comportant des substitutions aluminium en couche tétraèdrique de formule générale :

$$Na_x[Si_{8-X}\ Al_x]_{IV}\ [Al_4]_{VI}\ O_{20}\ (OH)_4,\ y\ H_2O$$

Les chiffres romains IV et VI indiquent que les atomes concernés sont en couches tétraèdrique ou octaédrique. Le mode de préparation est le suivant :

On dissout 53 x grammes de carbonate de sodium $Na_2\ CO_3$ dans la quantité d'eau juste nécessaire pour obtenir la solubilisation. On ajoute de l'acide nitrique concentré $HNO_3$ jusqu'à l'arrêt du

dégagement du dioxyde de carbone $CO_2$. On ajoute ensuite 374,724 (4 + x) grammes de nitrate d'aluminium Al $(NO_3)_3$, $9H_2O$, en solution dans la quantité d'eau juste nécessaire pour obtenir la solubilisation. Avant d'ajouter 208,33 (8 — x) grammes de tétraéthylorthosilicate à 0,288 gramme de silice Si $O_2$ par gramme de tétraéthylorthosilicate, on ajoute de l'éthanol en volume à peu près égal au volume de tétraéthylorthosilicate et au volume d'eau déjà présent. Après ajout du tétraéthylorthosilicate, on ajoute de l'ammoniaque $NH_4OH$. On obtient ainsi un gel qu'on laisse reposer 12 heures. On sèche à 70 °C pendant 24 heures, de façon à éliminer l'eau, l'alcool et l'ammoniac. Le produit sec est broyé, puis calciné à 350 °C, pendant 15 heures, de façon à décomposer les nitrates.

On prend ensuite une certaine masse M du gel obtenu, que l'on chauffe en autoclave dans un tube en or, à 340 °C, en présence d'un certain volume S d'une solution de soude NaOH d'une concentration C donnée pendant une durée comprise entre 2 et 25 jours.

La pâte obtenue est séchée à 80 °C pendant 5 heures. Elle est ensuite broyée puis redispersée dans de l'eau distillée à une concentration de 20 grammes par litre.

On a ainsi préparé sept argiles en faisant varier x, M, S et C.

Les conditions opératoires et les caractéristiques des produits obtenus sont données dans le Tableau I ci-après :

Tableau I

| Argile | Coefficient x | Masse de gel M en grammes | Volume de solution de soude S en ml | Concentration C de la solution de soude | Capacité d'échange cationique en milli-équivalent/100 g (1) | Surface spécifique en m²/g (2) |
|---|---|---|---|---|---|---|
| AST1 | 0,67 | 6,22 | 19,65 | 0,12 | 97 | 82 |
| AST2 | 0,67 | 6,38 | 20,85 | 0,13 | 101 | 85 |
| AST3 | 0,67 | 6,39 | 20,33 | 0,127 | 97 | 75 |
| AST4 | 0,67 | 6,01 | 20,78 | 0,105 | 96 | 80 |
| AST5 | 0,67 | 5,21 | 19,98 | 0,126 | 108 | 85 |
| AST6 | 0,67 | 100 | 250 | 0,15 | 94 | 75 |
| AST7 | 0,40 | 25 | 62,5 | 0,15 | 75 | non déterminée. |

(1) La capacité d'échange cationique est mesurée de la façon suivante : on échange les ions sodium $Na^+$ par des ions ammonium $NI_4^+$ à partir d'une solution d'acétate d'ammonium molaire. On lave à l'eau puis on attaque l'argile par de la soude. On obtient un dégagement d'ammoniac qui est reçu dans de l'acide borique. L'ammoniac fixé est dosé par de l'acide sulfurique.

(2) Mesurée par la méthode B.E.T.

Préparation de zéolites à structure bidimensionnelle selon l'invention, de la montmorillonite pontée, de l'hectorite pontée et de la laponite pontée.

On part des argiles suivantes :

a) des argiles à substitutions en couche tétraèdrique AST1, AST2, AST3, AST4, AST5, AST6 et AST7, préparés comme décrit précédemment,

b) d'une argile naturelle de Taïwan à substitution en couche tétraèdrique de formule :

$$Mg_{0,08}Ca_{0,08}Na_{0,52}K_{0,24}[Si_{7,38}Al_{0,62}]\ ^{IV}[Al_{3,12}Fe_{0,54}Mg_{0,34}]_{VI}O_{20}(OH)_4$$

La capacité d'échange cationique de cette argile est de 140 milliéquivalents/100 grammes. On a vérifié, par analyse infrarouge, que cette argile a des substitutions en couche tétraèdrique,

c) d'une hectorite, qui est une smectite naturelle de Californie, de formule générale :

$$yNa[Si_8]_{IV}[Mg_{6-y}Liy]_{VI}O_{20}[OH, F]_4$$

Cette hectorite ne contient pratiquement pas d'aluminium.

On a vérifié, par spectroscopie infrarouge, que cette hectorite ne contient pas de substitution en couche tétraèdrique,

d) d'une laponite, qui est une hectorite de synthèse de formule voisine.

On a vérifié, par analyse infrarouge, que cette hectorite ne contient pas de substitution en couche tétraèdrique.

e) d'une montmorillonite du Wyoming, qui est une smectite naturelle ne contenant pratiquement pas de subtitution en couche tétraèdrique (vérifié par spectroscopie infrarouge) et dont la surface spécifique externe à l'état sec est de 15 $m^2$/gramme.

Toutes ces argiles sont mises en suspension dans de l'eau. La fraction de ces argiles, dont la granulométrie est inférieure ou égale à 2 microns est séparée par décantation, la fraction dont la granulométrie est supérieure à 2 microns étant éliminée.

On prépare à partir de ces fractions d'argile des suspensions aqueuses d'argile dont la composition et les références sont données dans le tableau II ci-après :

Tableau II

| Argile de départ | Suspension | % en poids d'argile dans la suspension |
|---|---|---|
| AST 1 | SA 1 | 2 |
| AST 2 | SA 2 | 2 |
| AST 3 | SA 3 | 2 |
| AST 4 | SA 4 | 2 |
| AST 5 | SA 5 | 2 |
| AST 6 | SA 6 | 4 |
| AST 7 | SA 7 | 4 |
| Argile de Taïwan | SA 8 | 2 |
| Hectorite | SH | 4 |
| Laponite | SL | 4 |
| Montmorillonite | SM | 4 |

On prépare également :

des solutions I aqueuses de nitrate d'aluminium Al $(NO_3)_3$, $9H_2O$ de concentrations molaires variables,

des solutions II aqueuses de soude de concentrations molaires variables.

On ajoute une solution I à une solution II en quantités variables, sous agitation continue.

On obtient ainsi des solutions d'hydroxyde d'aluminium ayant des rapports $OH^-/Al^{3+}$ différents.

On prépare ensuite différents mélanges, à partir des solutions d'hydroxydes d'aluminium et des suspensions d'argile.

Les mélanges obtenus sont :

a) soit placés, en vue d'une dialyse, à une température de 20 °C, dans une poche composée d'une membrane d'acétate de cellulose semi-perméable, d'une porosité moyenne de 24 Angströms, la poche étant placée dans de l'eau déminéralisée pendant 24 heures et l'opération étant renouvelée quatre fois,

b) soit additionnés d'ammoniac concentré jusqu'à l'obtention d'un pH 5 et lavés avec de l'eau déminéralisée.

Les mélanges dialysés ou lavés sont centrifugés et séchés par lyophilisation.

On obtient ainsi :

à partir des argiles comportant des substitutions en couche tétraèdrique, les précurseurs des zéolites bidimensionnelles,

à partir des argiles ne comportant pas de substitution en couche tétraèdrique, des argiles pontées séchées.

On prélève, sur certains des produits séchés, des échantillons, qui sont examinés :

par diffraction de rayons X,

par résonance magnétique nucléaire pour certains.

Les produits séchés sont ensuite calcinés à 400 °C pendant 2 heures.

Les conditions de préparation de ces produits et leurs caractéristiques physiques sont données dans le tableau III ci-après :

Tableau III

| Argile de départ | Suspension utilisée | Référence du produit séché | Référence du produit calciné | Rapport $\frac{OH^-}{Al^{3+}}$ dans la solution d'hydroxyde | Solution I de nitrate d'aluminium utilisée | | Solution II de soude utilisée | | Volume de suspension utilisé en ml | Quantité d'aluminium en millimoles par g d'argile |
|---|---|---|---|---|---|---|---|---|---|---|
| | | | | | Volume en ml | Concentration | Volume en ml | Concentration | | |
| AST 1 | SA 1 | PZ 11 | ZSB11 | 1,2 | 25 | 0,4 M | 50 | 0,24 M | 50 | 10 |
| AST 1 | SA 1 | PZ 12 | ZSB12 | 1,4 | 25 | 0,4 M | 50 | 0,28 M | 50 | 10 |
| AST 2 | SA 2 | PZ 21 | ZSB21 | 1,2 | 25 | 0,4 M | 50 | 0,24 M | 50 | 10 |
| AST 2 | SA 2 | PZ 22 | ZSB22 | 1,4 | 25 | 0,4 M | 50 | 0,28 M | 50 | 10 |
| AST 3 | SA 3 | PZ 31 | ZSB31 | 1,2 | 25 | 0,4 M | 50 | 0,24 M | 50 | 10 |
| AST 3 | SA 3 | PZ 32 | ZSB32 | 1,4 | 25 | 0,4 M | 50 | 0,28 M | 50 | 10 |
| AST 4 | SA 4 | PZ 41 | ZSB41 | 1,2 | 25 | 0,4 M | 50 | 0,24 M | 50 | 10 |
| AST 4 | SA 4 | PZ 42 | ZSB42 | 1,4 | 25 | 0,4 M | 50 | 0,28 M | 50 | 10 |
| AST 5 | SA 5 | PZ 51 | ZSB51 | 1,2 | 25 | 0,4 M | 50 | 0,24 M | 50 | 10 |
| AST 5 | SA 5 | PZ 52 | ZSB52 | 1,4 | 25 | 0,4 M | 50 | 0,28 M | 50 | 10 |
| AST 6 | SA 6 | PZ 61 | ZSB61 | 1,2 | 66 | 0,2 M | 16,6 | 0,5 M | 50 | 6,66 |
| AST 7 | SA 7 | PZ 71 | ZSB71 | 1,2 | 66 | 0,2 M | 16,6 | 0,5 M | 50 | 6,66 |
| Argile de Taïwan | SA 8 | PZ 81 | ZSB81 | 1,2 | 25 | 0,4 M | 50 | 0,24 M | 50 | 10 |
| Hectorite | SH | HPS | HPT | 1,2 | 66 | 0,2 M | 16,6 | 0,5 M | 50 | 6,66 |
| Laponite | SL | LPS | LPT | 1,2 | 25 | 0,4 M | 50 | 0,24 M | 50 | 10 |
| Montmorillonite | SM | MPS | MPT | 1,2 | 66 | 0,2 M | 16,6 | 0,5 M | 50 | 6,66 |

Tableau III (suite)

| Caractéristiques des produits obtenus | | | | |
|---|---|---|---|---|
| Espacement basal en Angströms | | | Surface spécifique en $m^2/g$ | Volume poreux $cm^3/g$ |
| Argile de départ | Produit séché à température ambiante | Produit calciné à 400 °C | | |
| AST1 | 18,5 | 17,2 | 280 | 0,35 |
| AST1 | 18,5 | 17,5 | 285 | 0,36 |
| AST2 | 18,6 | 17,5 | 286 | non mesuré |
| AST2 | 18,2 | 17,5 | 277 | 0,36 |
| AST3 | 18,1 | 17,3 | 284 | non mesuré |
| AST3 | 18,2 | 17,6 | 282 | 0,28 |
| AST4 | 18 | 16,3 | 287 | 0,35 |
| AST4 | 17,9 | 16,6 | 271 | 0,35 |
| AST5 | 18 | 17 | 290 | 0,36 |
| AST5 | 18,1 | 17 | 290 | non mesuré |
| AST6 | 18 | 17,1 | 230 | non mesuré |
| AST7 | 18,2 | 17 | 255 | non mesuré |
| Argile de Taïwan | 18 | 17,3 | non mesurée | non mesurée |
| Hectorite | 18,5 | 17,6 | 205 | non mesuré |
| Laponite | 18 | 17 | non mesuré | non mesuré |
| Montmo-tillonite | 18,5 | 17,3 | 220 | non mesuré |

La dialyse du mélange suspension d'argile-solution d'hydroxyde a été utilisée dans toutes les préparations, sauf dans la préparation de la zéolite ZSB 61.

On constate, d'après le tableau III, que les zéolites à structure bidimensionnelle ZSB selon l'invention possèdent un bon espacement basal.

On a étudié par résonance magnétique nucléaire :

l'argile à substitution tétraèdrique AST 5, le précurseur PZ 51 de la zéolite à structure bidimensionnelle ZSB 51 et cette zéolite elle-même.

l'hectorite pontée séchée HPS et l'hectorite pontée témoin HPT.

la laponite pontée séchée LPS et la laponite pontée témoin LPT.

On a utilisé pour cette étude deux spectromètres composés d'aimants supraconducteurs d'inductions magnétiques de 8,45 tesla pour la noyau $^{29}Si$ et 11,7 tesla pour $^{27}Al$ et des systèmes à transformer de Fourier décrits par MEADOWS M.D. et AL., dans l'article intitulé : « High resolution solid-state NMR of quadrupolar Nuclei », paru dans PROCEEDINGS OF NATIONAL US ACADEMY OF SCIENCES, Volume 79, pages 1 351 à 1 355 (1982). Ces deux spectromètres sont équipés de systèmes d'acquisition de données. Le système utilisé pour faire tourner l'échantillon autour de l'angle magique est celui décrit par ANDREWS E.R. dans l'article intitulé « The narrowing of NMR spectra of solids by high-speed specimen rotation and the resolution of chemical shift and spin multiplet structures of solids », paru dans PROGRESS IN NMR SPECTROSCOPY, Numéro 8, pages 1 à 39 (1971).

Les déplacements chimiques, par rapport à des références, sont exprimés en parties par million de la fréquence utilisée pour provoquer les transitions. On a utilisé comme référence pour le noyau $^{27}Al$ une solution aqueuse molaire de trichlorure d'aluminium et pour le noyau $^{29}Si$ du tétraméthylsilane.

Les déplacements chimiques sont fonction de l'environnement des atomes étudiés (silicium et aluminium).

On a représenté sur les figures 1 à 4 jointes à la présente description les spectres des produits étudiés.

Les figures 1 et 2 correspondent au noyau $^{29}Si$ et les figures 3 et 4 au noyau $^{27}Al$.

Les fréquences utilisées lors des analyses sont respectivement de 71,52 Mégahertz pour le silicium et de 130,3 Mégahertz pour l'aluminium.

L'intégration du signal permet de doser les différentes espèces d'atomes de silicium et d'aluminium comportant des environnements différents.

Dans le cas de séparations non évidentes, des déconvolutions mathématiques ont été effectuées, de manière à doser les différentes espèces d'atomes présentes.

Dans le tableau IV, ci-après, sont indiqués les déplacements δ en p.p.m. des raies de résonance des noyaux $^{29}Si$ et les contributions des différentes espèces d'atomes de silicium à cette raie (en % de la surface totale de la raie).

Tableau IV

| Produit | 1ère raie | | 2ème raie | | 3ème raie | |
|---|---|---|---|---|---|---|
| | % | - δ | % | - δ | % | - δ |
| AST 5 | 66,7 | 95,2 | 30 | 90,2 | 3,3 | 85,6 |
| PZ 51 | 63,4 | 95,9 | 31,2 | 91,2 | 5,4 | 87 |
| ZSB 51 | 65,8 | 96,2 | 28,2 | 92,4 | 6 | 86,6 |
| HPS | 100 | 97,9 | - | - | - | - |
| HPT | 100 | 98 | - | - | - | - |
| LPS | 100 | 95,5 | - | - | - | - |
| LPT | 100 | 96 | - | - | - | - |

La première raie correspond aux atomes de silicium ne comportant que des atomes de silicium dans leur environnement.

La deuxième raie correspond aux atomes de silicium comportant un atome d'aluminium dans leur environnement.

La troisième raie correspond aux atomes de silicium comportant deux atomes d'aluminium dans leur environnement.

Pour les argiles pontées témoin, il n'y a qu'une raie et, lors de la calcination, aucune déformation de celle-ci n'est observable.

Pour les produits obtenus à partir d'argile à substitution tétraèdrique, la raie est composite et on note une déformation au cours de la calcination, ce qui traduit l'apparition d'un produit nouveau.

Dans le tableau V ci-après, sont indiqués les δ en p.p.m. des raies de résonance des noyaux $^{27}Al$ et les contributions des différentes espèces d'atomes d'aluminium à cette raie (en % de la surface totale de la raie).

Tableau V

| Produit | 1ère raie | | 2ème raie | | 3ème raie | |
|---|---|---|---|---|---|---|
| | % | δ | % | δ | % | δ |
| AST 5 | 24 | 69,3 | - | - | 76 | 3 |
| PZ 51 | 22 | 69,1 | 6 | 62,3 | 72 | 3,4 |
| ZSB 51 | 10 | 67,5 | 4,5 | 56,5 | 85,5 | 2,6 |
| HPS | - | - | 10 | 61 | 90 | 6,8 |
| HPT | - | - | 18,5 | 59,1 | 81,5 | 5,9 |
| LPS | - | - | 21 | 59,3 | 79 | 7,1 |
| LPT | - | - | 37 | 63,5 | 63 | 4,8 |

La première raie correspond aux atomes d'aluminium des tétraèdres des feuillets de l'argile. Elle n'existe, comme on peut le constater sur la figure 3 et dans le tableau V, que pour les argiles à substitution aluminium en couche tétraèdrique modifiées ou non par un traitement par un hydroxyde d'aluminium.

La deuxième raie correspond aux atomes d'aluminium de nouveaux tétraèdres formés lors du traitement de l'argile par un hydroxyde d'aluminium. Les atomes d'aluminium de ces tétraèdres proviennent soit de l'oligomère aluminique servant au traitement de l'argile, soit de tétraèdres des feuillets de l'argile dont l'environnement a été modifié. Elle n'existe pas pour les argiles non traitées par l'hydroxyde d'aluminium.

La troisième raie correspond aux atomes d'aluminium des octaèdres de l'argile de départ et des octaèdres formés lors du traitement de l'argile par un hydroxyde d'aluminium.

Pour les argiles pontées témoin, on n'observe pas de modification de la deuxième raie au cours de la calcination. Par contre, pour la zéolite ZSB 51, la raie est à 56,5 p.p.m., alors que, dans le précurseur PZ 51, la raie est à 62,3, ce qui montre bien l'apparition d'un produit nouveau au cours de la calcination.

Exemple 2

Cet exemple concerne des essais d'hydroisomérisation et d'hydrocraquage d'une charge de normal heptane à l'aide de catalyseurs préparés à partir de zéolites à structure bidimensionnelle selon l'invention et d'argiles pontées témoin préparées selon l'exemple 1.

A titre de comparaison, on a également préparé un catalyseur à partir d'une zéolite ultrastable Y (ZUSY). Cette zéolite ultrastable Y est préparée en faisant subir à une zéolite NaY un traitement d'échange par de l'acétate d'ammonium. La zéolite échangée est ensuite traitée à 650 °C par de la vapeur d'eau. Le catalyseur préparé à partir de la zéolite ultrastable Y est considéré par l'homme de l'art comme une référence dans la réaction catalytique indiquée ci-dessus.

Les catalyseurs sont préparés de la façon suivante :

On imprègne la zéolite à structure bidimensionnelle ou l'argile pontée utilisée comme support à l'aide d'une quantité suffisante d'une solution aqueuse de chlorure de platine tétramine Pt $(NH_3)_4Cl_2$ de façon à déposer 1 % en poids de platine sur le support.

Après séchage à 110 °C pendant 14 heures, on calcine à l'air à 400 °C pendant 2 heures les produits obtenus. On les réduit ensuite à 400 °C pendant 2 heures par un courant d'hydrogène. On obtient ainsi les catalyseurs.

On place dans un réacteur 200 milligrammes de catalyseur. On introduit le normal heptane et de l'hydrogène dans un rapport volumique hydrogène/heptane de 16. Le débit de normal heptane est de 1 gramme par gramme de catalyseur par heure.

On élève la température par programmation linéaire de 150 à 400 °C, la pression étant la pression atmosphérique.

On analyse les effluents à la sortie du réacteur par chromatographie en phase gazeuse. On obtient notamment les produits suivants :

Isomères du normal heptane :

2 méthyl hexane
3 méthyl hexane

3 éthyl pentane
2,3 méthyl pentane
2,4 méthyl pentane
2,2 méthyl pentane
3,3 méthyl pentane.

Produits d'hydrocraquage et d'hydrogénolyse :

éthane, méthane, propane
isobutane, normal butane
2, méthyl butane
normal pentane
2,3 méthyl butane
2, méthyl pentane
3, méthyl pentane
normal hexane.

On donne dans le tableau VI ci-après les résultats des essais effectués.
Pour chaque catalyseur figurent dans ce tableau :
la température à laquelle 20 % du normal-heptane est converti, c'est-à-dire transformé en isomères et en produits de craquage,
la température à laquelle on obtient un maximum de produits isomérisés,
la valeur du rapport produits isomérisés/normal heptane converti à la température du maximum d'isomérisation, ce rapport représentant la sélectivité du catalyseur,
la conversion à la température du maximum d'isomérisation.

Tableau VI

| Support | Catalyseur | Température pour 20 % de conversion en °C | Température en °C du maximum d'isomérisation. | Rapport produits isomérisés / normal heptane converti à la température du maximum d'isomérisation | Conversion en % au maximum d'isomérisation |
|---|---|---|---|---|---|
| ZSB 31 | CZSB 31 | 208 | 255 | 80,5 | 82 |
| ZSB 32 | CZSB 32 | 210 | 258 | 84 | 75 |
| ZSB 41 | CZSB 41 | 209 | 260 | 87 | 85 |
| ZSB 42 | CZSB 42 | 204 | 260 | 88 | 72 |
| ZSB 51 | CZSB 51 | 205 | 258 | 87,2 | 75 |
| ZSB 52 | CZSB 52 | 204 | 259 | 89,5 | 76 |
| ZSB 61 | CZSB 61 | 205 | 260 | 84,5 | 83 |
| ZSB 62 | CZSB 62 | 205 | 262 | 86,5 | 81 |
| HPT | CHPT | 303 | 355 | 40 | 53 |
| MPT | CMPT | 308 | 365 | 63 | 46 |
| ZUSY | CZ USY | 204 | 225 | 72 | 65 |

On a reporté, sur la figure 5, les courbes des pourcentages de produits isomérisés en fonction des pourcentages de produits de craquage pour les catalyseurs préparés à partir de zéolites selon l'invention CZSB 41, CZSB 42, CZSB 52 et CZSB 61, des catalyseurs témoin CHPT, CMPT et CZUSY.

On constate, d'après le tableau VI et la courbe, que les catalyseurs CZSB préparés à partir des zéolites à structure bidimensionnelle ZSB selon l'invention possèdent des propriétés isomérisantes bien supérieures à celles des catalyseurs préparés à partir d'argiles pontées et ce à des températures beaucoup plus basses. D'après la courbe, on voit en particulier que le maximum d'isomérisation obtenu par un catalyseur préparé à partir d'une argile pontée est de l'ordre de 35 % à comparer avec les 75 % environ des catalyseurs préparés selon l'invention.

D'après le tableau VI, on voit encore qu'avec le catalyseur préparé à partir de la zéolite ultrastable Y, on obtient au maximum d'isomérisation 72 % de produits isomérisés dans les produits de conversion alors qu'avec les zéolites selon l'invention on obtient toujours plus de 80 %.

Les catalyseurs préparés à partir des zéolites bidimensionnelles selon l'invention sont donc de très bons catalyseurs d'hydroisomérisation.

Exemple 3

Cet exemple concerne des essais d'hydroisomérisation et d'hydrocraquage d'une charge de normal décane à l'aide de catalyseurs préparés à partir de zéolites à structure bidimensionnelle selon l'invention et d'une argile pontée témoin préparées selon l'exemple 1.

On prépare des catalyseurs avec 1 % en poids de platine de la même façon que dans l'exemple 2.

On place dans un réacteur 0,6 millilitre de catalyseur. On fait alors passer sur le catalyseur un mélange de normal-décane et d'hydrogène à la pression atmosphérique, avec un débit $F_0$ de normal-décane en tête du réacteur tel que, W étant le poids de catalyseur, le rapport W/F est égal à 518 kg-seconde/mole, ce qui correspond à 0,7 g de normal-décane par gramme de catalyseur par heure, le rapport hydrogène/normal-décane étant égal à71 normaux litres/litre.

On élève la température par paliers de 100 à 300 °C.

On analyse les effluents à la sortie du réacteur par chromatographie en phase gazeuse.

On obtient notamment les produits suivants :

Isomères du normal-décane ou produits d'hydroisomérisation

méthylnonanes
éthyl octanes
propyl heptanes
diméthyloctanes

Produits de craquage et d'hydrogénolyse

méthane
éthane
propane
butanes
pentanes
hexanes
heptanes

On donne, dans le tableau VII ci-après, les résultats des essais effectués.

Pour chaque catalyseur figurent dans ce tableau :

la température à laquelle 20 % du normal-décane est converti, c'est-à-dire transformé en isomères et en produits de craquage,

la température à laquelle on obtient un maximum de produits isomérisés,

la valeur du rapport produits isomérisés/normal-décane converti à la température du maximum d'isomérisation,

la conversion à la température du maximum d'isomérisation.

Tableau VII

| Support | Catalyseur | Température pour 20 % conversion en °C | Température en °C du maximum d'isomérisation | Produits isomérisés / normal décane converti à la température du maximum d'isomérisation | Convertion en % au maximum d'isomérisation |
|---|---|---|---|---|---|
| ZSB 11 | CZSB 11 | 185 | 222 | 80 | 80 |
| ZSB 12 | CZSB 12 | 176 | 231 | 82 | 89 |
| ZSB 21 | CZSB 21 | 185 | 222 | 83 | 85 |
| ZSB 22 | CZSB 22 | 176 | 218 | 85 | 90 |
| ZSB 81 | CZSB 81 | 177 | 215 | 67 | 85 |
| MPT | CMPT | 230 | 281 | 70 | 74 |

On constate d'après le tableau VII qu'avec les catalyseurs selon l'invention, on obtient toujours un maximum d'isomérisation à une température beaucoup plus basse et, dans la plupart des cas, ce maximum est beaucoup plus élevé.

Exemple 4

Cet exemple concerne des essais de craquage d'une charge de cumène à l'aide de catalyseurs constitués par des zéolites à structure bidimensionnelle selon l'invention, et d'argiles pontées témoin préparées selon l'exemple 1.

Les zéolites à structure bidimensionnelle et les argiles pontées sont calcinées à l'air pendant 2 heures à 400 °C. On place dans un réacteur 100 mg de catalyseur. On introduit dans le réacteur du cumène à un débit de 2 grammes de cumène par gramme de catalyseur et par heure. On élève la température de façon linéaire jusqu'à 450 °C.

On analyse les effluents à la sortie du réacteur par chromatographie en phase gazeuse. Le cumène est craqué en propylène et en benzène.

On donne dans le tableau VIII ci-après la conversion du cumène à 300 °C selon les différents catalyseurs employés.

Tableau VIII

| Catalyseur | Conversion en % en poids à 300 °C |
|---|---|
| ZSB 31 | 54 |
| ZSB 32 | 47 |
| ZSB 41 | 61 |
| HPT | 4 |
| MPT | 5 |

Ce tableau montre la supériorité des zéolites bidimensionnelles selon l'invention sur les argiles pontées témoins dans le craquage du cumène.

Exemple 5

Cet exemple concerne des essais de craquage catalytique de charges d'hydrocarbures provenant de la distillation du pétrole brut à l'aide de catalyseurs constitués par la zéolite bidimensionnelle selon l'invention ZSB 31 et de l'argile pontic témoin MPT, préparées selon l'exemple 1.

Préalablement aux essais catalytiques proprement dits, les catalyseurs ont subi, dans certains cas, un traitement de mise à l'équilibre des catalyseurs.

Traitement de mise à l'équilibre des catalyseurs

Ce traitement a pour but de mettre le catalyseur dans l'état dans lequel il se trouve dans une unité de craquage. Dans une unité, en effet, le catalyseur vieillit du fait que, pendant la régénération, il se trouve en présence de vapeur d'eau, à température élevée. Une bonne partie de la zéolite est alors détruite.

Le traitement A consiste à mettre le catalyseur dans une atmosphère de vapeur d'eau, à haute température.

Essais catalytiques

Les essais catalytiques ont été effectués avec deux charges d'hydrocarbures :

La charge 1 est constituée par un distillat provenant de la distillation sous pression réduite du résidu de la distillation sous pression atmosphérique d'un pétrole brut ARAMCO.

Les caractéristiques de cette charge sont les suivantes :

masse volumique à 15 °C (mesurée selon la norme AFNOR NFT 60-101) : 930 kg/m$^3$,
viscosité à 100 °C (mesurée selon la norme AFNOR NFT 60-100) : 9,8 centistokes,
soufre (mesuré par dosage chimique) : 2,8 % en poids,
nickel (mesuré par absorption atomique) : 0,1 p.p.m.,
vanadium (mesuré par absorption atomique) : 0,25 p.p.m.,

résidu « CONRADSON » (mesuré selon la norme AFNOR NFT 60-116) : 0,9 % en poids,
indice de réfraction : 1,506.

La charge 2 est constituée par un résidu provenant de la distillation sous pression atmosphérique d'un pétrole brut américain.

Les caractéristiques de cette charge sont les suivantes :
masse volumique à 15 °C (mesurée selon la norme AFNOR NFT 60-101) : 903 kg/$m^3$,
viscosité à 100 °C (mesurée selon la norme AFNOR NFT 60-100) : 12,8 centistokes,
soufre (mesuré par dosage chimique) : 0,45 % en poids,
nickel (mesuré par absorption atomique) : 11 p.p.m.,
vanadium (mesuré par absorption atomique) : 25 p.p.m.,
résidu « CONRADSON » (mesuré selon la norme AFNOR NFT 60-116) : 3,3 % en poids,
indice de réfaction : 1,492.

Les essais sont effectués dans un pilote de microactivité correspondant à la norme ASTM D 3907-80.

Les conditions opératoires sont les suivantes :
la température : 483 °C pour les essais T1, 1, T2, 2 et 530 °C pour l'essai 3,
poids de charge passant par heure pour une unité de poids de catalyseur : 16 pour les essais T1, 1, T2, 2 et 4,5 pour l'essai 3,
temps de réaction : 75 secondes pour les essais, T1, 1, T2, 2 et 15 secondes pour l'essai 3.

Les effluents sont recueillis à la sortie du réacteur et analysés par chromatographie. Le coke formé sur le catalyseur est déterminé par combustion.

Les résultats des essais figurent dans le tableau IX ci-après.

(Voir Tableau page 16)

Tableau IX

| ESSAI N° | | T1 | 1 | T2 | 2 | 3 |
|---|---|---|---|---|---|---|
| CATALYSEUR | | MPT | ZSB31 | MPT | ZSB31 | ZSB31 |
| Traitement de mise à l'équilibre | | sans | sans | 6 heures 20% d'eau 700 °C | 6 heures 20% d'eau 700 °C | 30 heures - 20 % d'eau 650 °C 30 heures - 0% d'eau 800 °C |
| CONVERSION en % en poids (1) | | 39 | 94,3 | 5 | 77,8 | 71,3 |
| PRODUITS FORMES (en % en poids) | Hydrogène | 0,3 | 0,3 | 2,1 | 0,3 | 0,7 |
| | Gaz (2) | 10,4 | 26,8 | 0,1 | 21,7 | 19,4 |
| | Essence (3) | 20,4 | 37,9 | 2,4 | 38,1 | 37,1 |
| | Gazole (4) | 16,1 | 5,1 | 5,8 | 16,4 | 22,6 |
| | Résidu (5) | 44,9 | 0,6 | 89,2 | 5,8 | 6,1 |
| | Coke | 7,9 | 29,3 | 0,4 | 17,7 | 14,1 |

(1) Pourcentage de la charge transformée.
(2) Hydrocarbures ayant de 1 à 4 atomes de carbone, gazeux à la température ambiante.
(3) Hydrocarbures ayant des points d'ébullition compris entre 25° et 215 °C.
(4) Hydrocarbures ayant des points d'ébullition compris entre 215 °C et 375 °C.
(5) Hydrocarbures ayant des points d'ébullition supérieurs à 375 °C.
Les essais T1, 1, T2 et 2 sont effectués avec la charge 1 et l'essai 3 avec la charge 3.

Ces essais montrent que les catalyseurs, selon l'invention, sont de bons catalyseurs de craquage catalytique, bien meilleurs que le catalyseur témoin puisque donnant plus de conversion en particulier en essence ce qui est un des buts de l'invention. De plus, le catalyseur témoin ne résiste pas au traitement de mise à l'équilibre.

## Revendications

1. Zéolite à structure bidimensionnelle, présentant un spectre distinctif de résonance magnétique nucléaire, obtenue par un procédé comportant les étapes suivantes :

a) le traitement d'une argile du type beidellite dans laquelle des atomes de silicium ont été remplacés par des atomes d'aluminium dans les couches tétraédriques, sous forme d'une suspension aqueuse, par une solution aqueuse contenant au moins un hydroxyde d'au moins un métal ;

b) l'élimination de l'excès de l'hydroxyde du métal ayant pas réagi sur l'argile, ladite élimination conduisant, après séchage de l'argile traitée, à l'obtention d'un précurseur de la zéolite ;

c) le traitement thermique du précurseur de la zéolite, ledit traitement conduisant à l'obtention de la zéolite à structure bidimensionnelle ;

caractérisée en ce que ledit traitement thermique du précurseur a été effectué à une température comprise entre 250 et 450 °C.

2. Zéolite à structure bidimensionnelle selon la revendication 1, caractérisée en ce que l'argile est choisie dans le groupe constitué par les argiles naturelles et synthétiques.

3. Zéolite à structure bidimensionnelle selon l'une des revendications 1 et 2, caractérisée en ce que l'hydroxyde métallique est choisi dans le groupe constitué par les hydroxydes de l'aluminium, du nickel, du cobalt, du molybdène, du rhénium, du fer, du cuivre, du ruthénium, du chrome, du lanthane, du cérium, du bore, du gallium, du titane ou les hydroxydes mixtes de ces éléments.

4. Zéolite à structure bidimensionnelle selon l'une des revendications 1 à 3, caractérisée en ce que l'hydroxyde métallique est un hydroxyde d'aluminium.

5. Utilisation de la zéolite à structure bidimensionnelle selon l'une des revendications 1 à 4 comme catalyseur ou comme support de catalyseur.

6. Utilisation selon la revendication 5 comme catalyseur ou comme support de catalyseur dans un procédé de conversion d'hydrocarbures, éventuellement en présence d'hydrogène.

7. Utilisation selon la revendication 6, dans un procédé où la réaction de conversion est une réaction d'isomérisation et/ou de craquage d'hydrocarbures.

8. Utilisation selon la revendication 6, dans un procédé où la réaction de conversion est une réaction de craquage catalytique d'une charge d'hydrocarbures, en particulier d'une charge constituée par le résidu de la distillation d'un pétrole brut.

## Claims

1. A zeolite with two-dimensional structure, having a characteristic nuclear magnetic resonance spectrum, obtained by a process comprising the following stages :

a) the treatment of a clay of beidelitte type, in which the silicon atoms have been replaced by aluminium atoms in the tetrahedral layers, in the form of an aqueous suspension, by an aqueous solution containing at least one hydroxide of at least one metal ;

b) the removal of the excess hydroxide of the metal which has not reacted on the clay, said removal resulting, after drying the treated clay, in the obtaining of a precursor of the zeolite :

c) the heat treatment of the precursor of the zeolite, said treatment resulting in the obtaining of a zeolite with two-dimensional structure ;

characterised in that the heat treatment of the precursor has been carried out at a temperature of between 250 and 450 °C.

2. A zeolite with two-dimensional structure according to claim 1, characterised in that the clay is chosen from the group comprising the natural and synthetic clays.

3. A zeolite with two-dimensional structure according to any of claims 1 and 2, characterised in that the metal hydroxide is chosen from the group comprising the hydroxides of aluminium, nikel, cobalt, molybdenum, rhenium, iron, copper, ruthenium, chromium, lanthanum, cerium, boron, gallium, titanium or mixed hydroxides of these elements.

4. A zeolite with two-dimensional structure according to any of claims 1 to 3, characterised in that the metallic hydroxide is an aluminium hydroxide.

5. Use of the zeolite with two-dimensional structure according to any of claims 1 to 4 as a catalyst or a catalyst carrier.

6. Use according to claim 5 as a catalyst or a catalyst carrier in a process for the conversion of hydrocarbons, optionally in the presence of hydrogen.

7. Use according to claim 6 in a process in which the conversion reaction is an isomerisation and/or hydrocarbon cracking reaction.

8. Use according to claim 6 in a process in which the conversion reaction is a reaction for the catalytic cracking of a hydrocarbon charge, in particular of a charge comprising the residue from the distillation of crude petroleum.

## Patentansprüche

1. Zeolith mit zweidimensionaler Struktur, welcher ein unterscheidendes magnetisches Kernresonanzspektrum aufweist und mittels eines Verfahrens mit den folgenden Schritten erhalten wurde :

a) Behandlung eines Tons vom Beidellit-Typ, bei dem Siliciumatome durch Aluminiumatome in den Tetraederschichten ersetzt worden sind, in Form einer wäßrigen Lösung, die mindestens ein Hydroxid mindestens eines Metalls enthält,

b) Eliminierung des überschüssigen Metallhydroxids, welches nicht mit dem ton reagiert hat, wobei die Eliminierung nach Trocknung des behandelten Tons zu einem Vorläufer des Zeoliths führt, und

c) thermische Behandlung des Zeolith-Vorläufers, welche zum Zeolithen mit zweidimensionaler Struktur führt,

dadurch gekennzeichnet, daß die thermische Behandlung des Vorläufers bei einer Temperatur zwischen 250 und 450 °C durchgeführt worden ist.

2. Zeolith mit zweidimensionaler Struktur nach Anspruch 1, dadurch gekennzeichnet, daß der Ton aus der Gruppe bestehend aus den natürlichen und synthetischen Tonen ausgewählt wird.

3. Zeolith mit zweidimensionaler Struktur nach Anspruch 1 oder 2, dadurch gekennzeichnet, daß das metallische Hydroxid aus der Gruppe bestehend aus den Hydroxiden des Aluminiums, des Nickels, des Kobalts, des Molybdäns, des Rheniums, des Eisens, des Kupfers, des Ruthemiums, des Chroms, des Lanthans, des Cers, des Bors, des Galliums, des Titans oder den gemischten Hydroxiden dieser Elemente ausgewählt wird.

4. Zeolith mit zweidimensionaler Struktur nach Anspruch 1, 2 oder 3, dadurch gekennzeichnet, daß es sich bei dem metallischen Hydroxid um Aluminiumhydroxid handelt.

5. Verwendung des Zeolithen mit zweidimensionaler Struktur nach einem der Ansprüche 1 bis 4 als Katalysator oder als Katalysatorträger.

6. Verwendung nach Anspruch 5 als Katalysator oder als Katalysatorträger bei einem Verfahren zur Umwandlung von Kohlenwasserstoffen, gegebenenfalls in Gegenwart von Wasserstoff.

7. Verwendung nach Anspruch 6 bei einem Verfahren, wo es sich bei der Umwandlungsreaktion um eine Reaktion zur Isomerisation und/oder zum Cracken von Kohlenwasserstoffen handelt.

8. Verwendung nach Anspruch 6 bei einem Verfahren, wo es sich bei der Umwandlungsreaktion um eine Reaktion zum katalytischen Cracken einer Charge von Kohlenwasserstoffen handelt, insbesondere einer Charge, welche aus dem Rückstand der Destillation eines Roherdöls besteht.

FIG.1

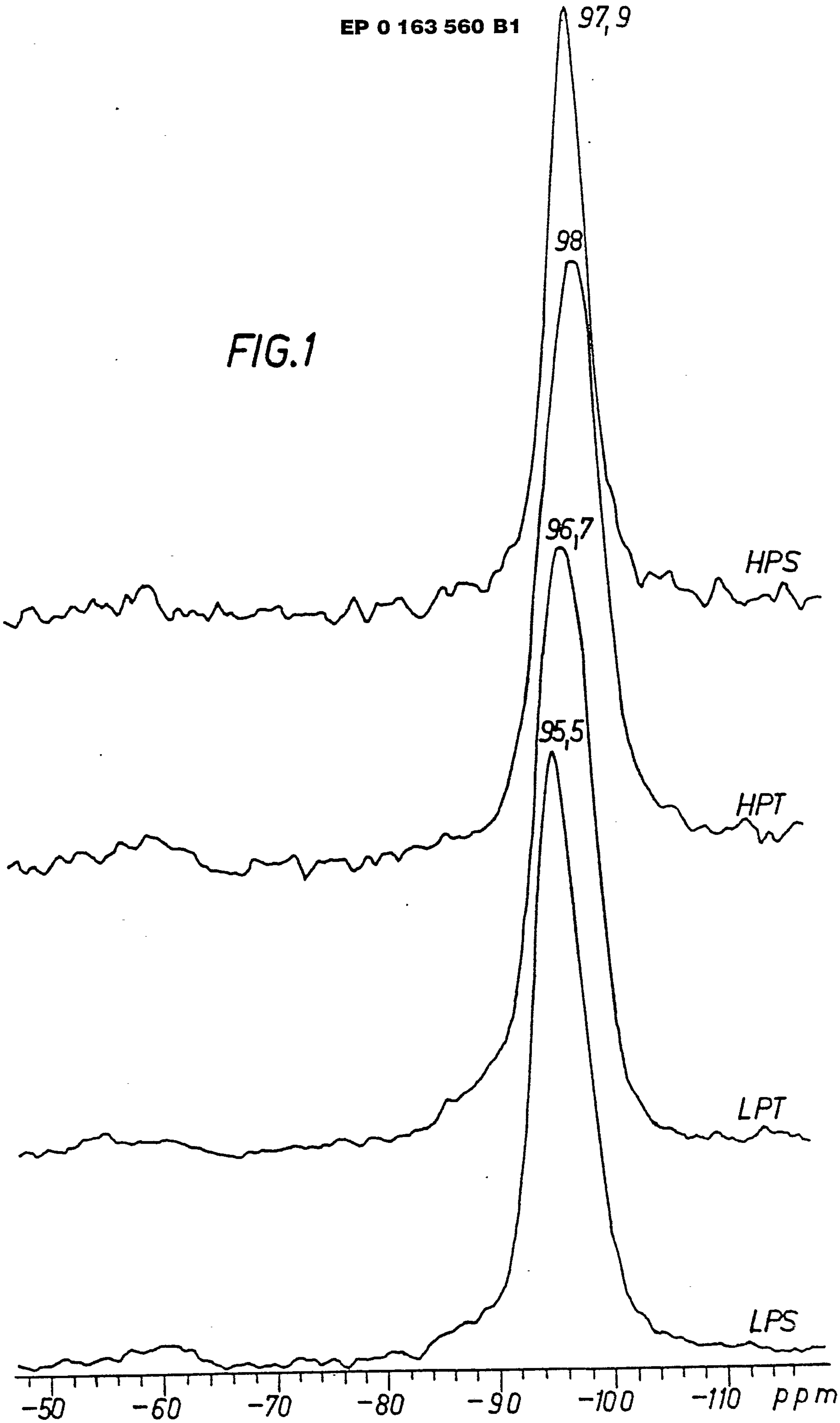

FIG.2

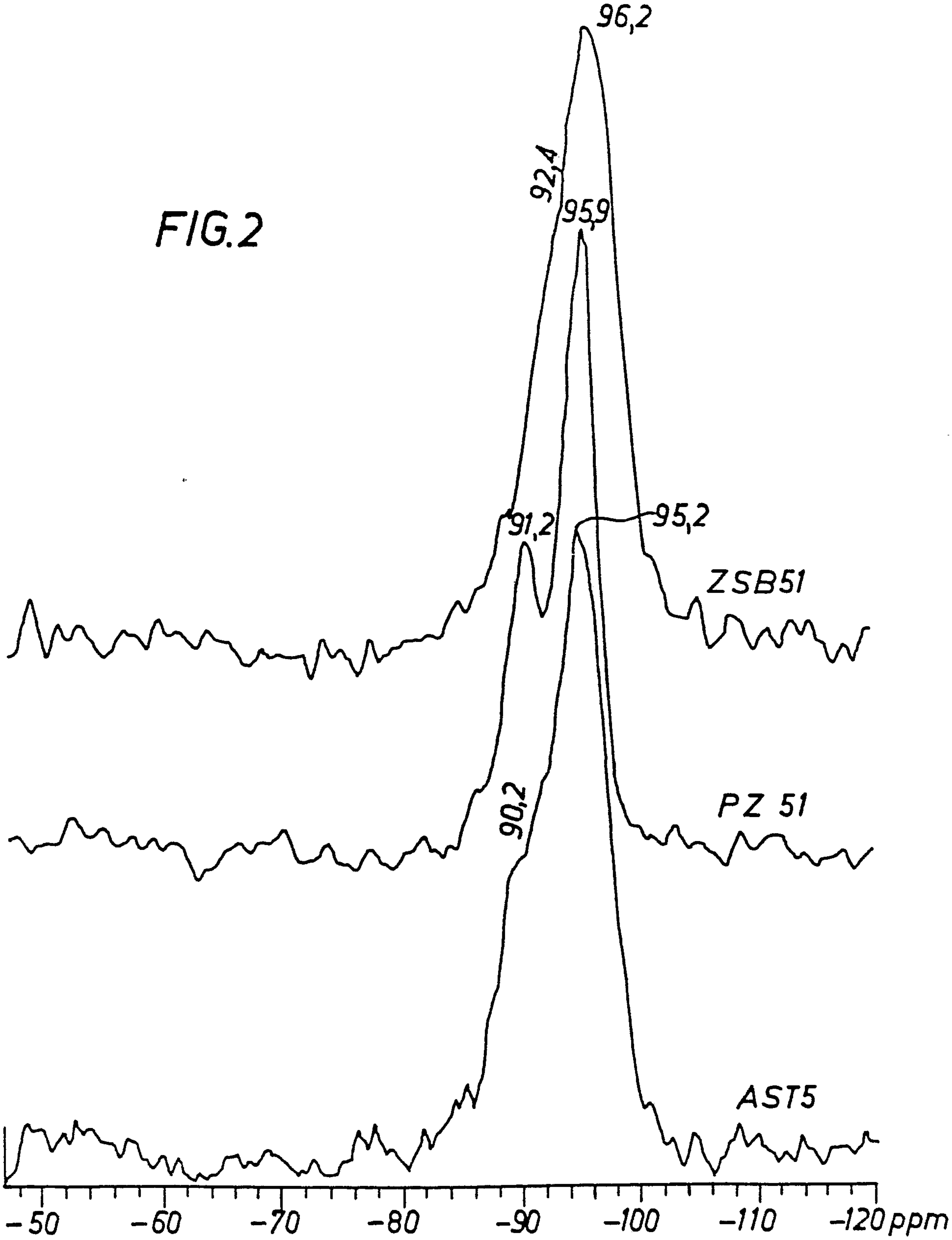
96,2
92,4
95,9
91,2
95,2
ZSB51
PZ 51
90,2
AST5
-50
-60
-70
-80
-90
-100
-110
-120 ppm

FIG.3

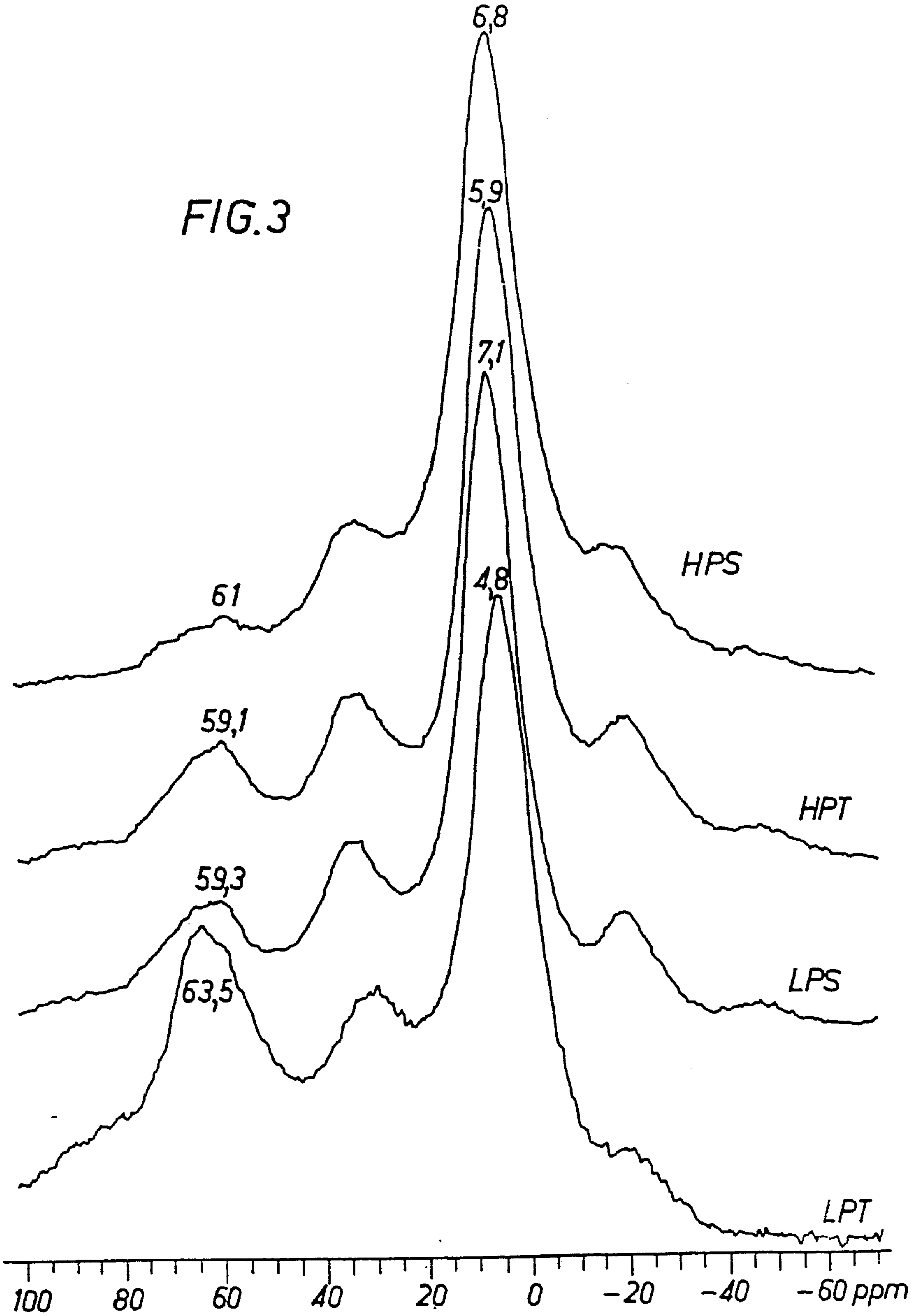
6,8
5,9
7,1
4,8
61
59,1
59,3
63,5
HPS
HPT
LPS
LPT
100
80
60
40
20
0
-20
-40
-60 ppm

FIG. 4

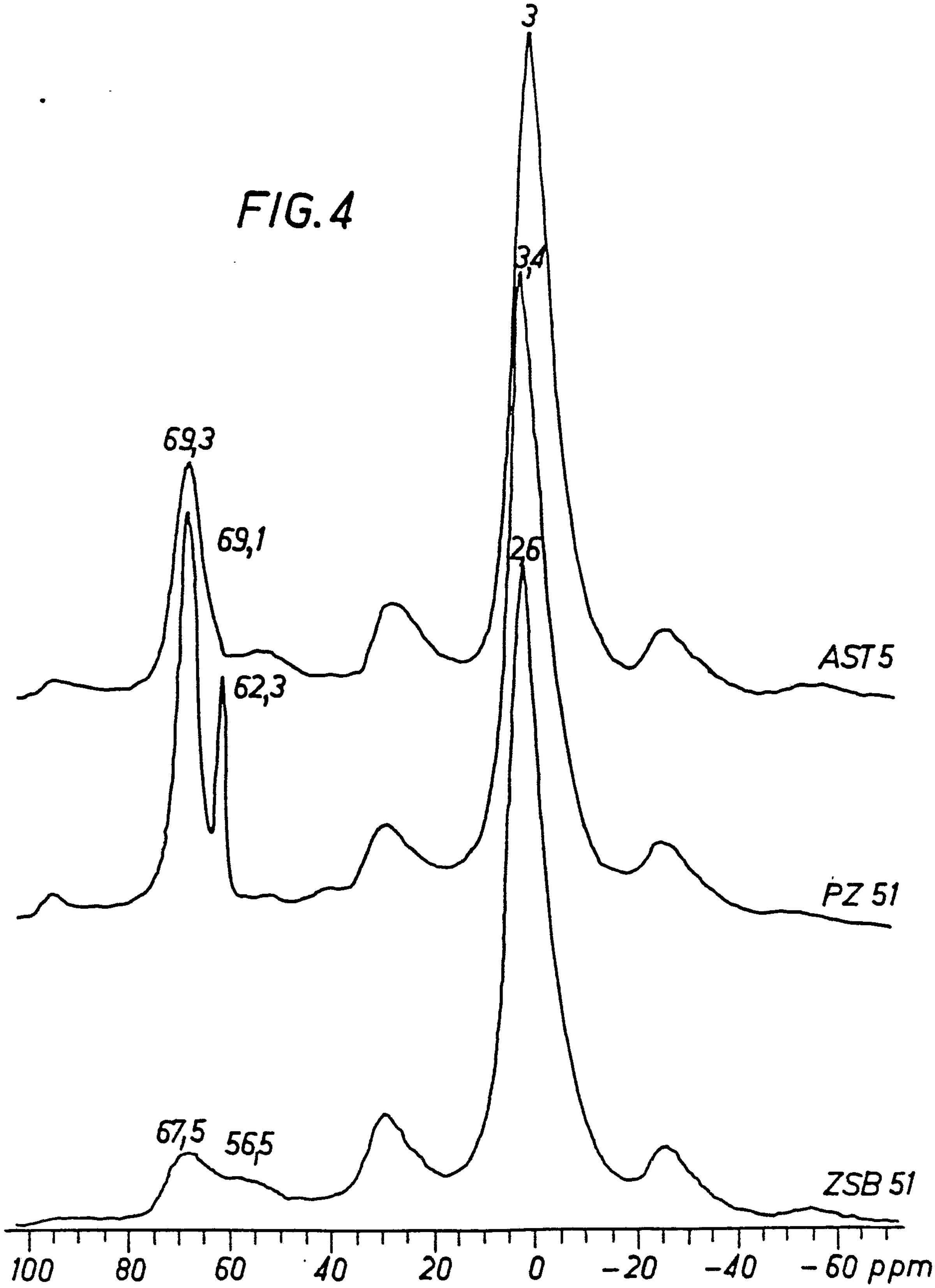
3
3,4
69,3
69,1
2,6
AST 5
62,3
PZ 51
67,5
56,5
ZSB 51
100
80
60
40
20
0
−20
−40
−60 ppm

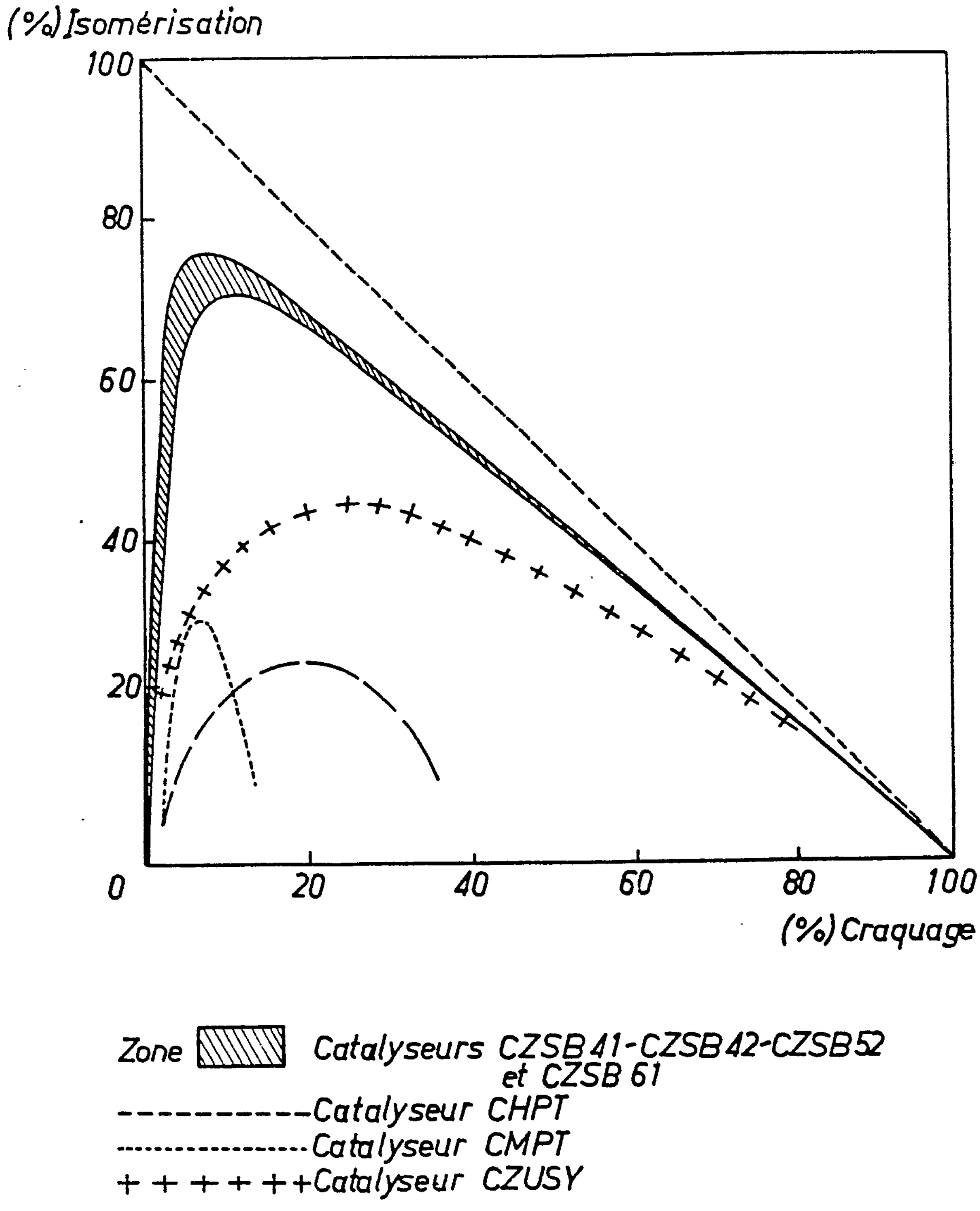
(%) Isomérisation
100
80
60
40
20
0
20
40
60
80
100
(%) Craquage
Zone
Catalyseurs CZSB 41-CZSB 42-CZSB 52 et CZSB 61
Catalyseur CHPT
Catalyseur CMPT
Catalyseur CZUSY

FIG. 5